(19)

Europäisches Patentamt
European Patent Office
Office européen des brevets

(11)  **EP 3 449 737 A1**

(12)  **EUROPEAN PATENT APPLICATION**
published in accordance with Art. 153(4) EPC

(43) Date of publication:
06.03.2019 Bulletin 2019/10

(21) Application number: 17789980.4

(22) Date of filing: 28.04.2017

(51) Int Cl.:
*A23L 33/105* *(2016.01)*    *C12J 1/04* *(2006.01)*
*A61K 8/97* *(2017.01)*    *A61Q 19/00* *(2006.01)*
*A61Q 19/08* *(2006.01)*

(86) International application number:
**PCT/KR2017/004609**

(87) International publication number:
**WO 2017/188796 (02.11.2017 Gazette 2017/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: 28.04.2016 KR 20160052476

(71) Applicant: **Sempio Foods Company**
**Seoul 04557 (KR)**

(72) Inventors:
• **BAEK, Eun Jong**
**Incheon 21920 (KR)**

• **JEONG, Gang Hee**
**Cheongju-si**
**Chungcheongbuk-do 28166 (KR)**
• **KIM, Kyung-Ok**
**Yongin-si**
**Gyeonggi-do 16822 (KR)**
• **CHOI, Yong Ho**
**Cheongju-si**
**Chungcheongbuk-do 28660 (KR)**
• **HURH, Byung-Serk**
**Sejong 30101 (KR)**

(74) Representative: **Alatis**
**109 Bd Haussmann**
**75008 Paris (FR)**

(54)  **FERMENTED APPLE PRODUCT CONTAINING GREAT QUANTITY OF CALCIUM ION**

(57)     An aspect of the present specification relates to a fermented fruit product that has an outstanding taste and flavor with a content of $Ca^{2+}$ ions in an amount of 100 ppm or higher on the basis of the total weight thereof, a composition includiing the same, and a preparation method thereof. In a particular embodiment, the fruit comprises an apple. The fermented fruit product has an outstanding taste and flavor and exhibits an excellent effect of enhancing the absorption of calcium into the body or through the skin.

FIG. 1

EP 3 449 737 A1

**Description**

[Technical Field]

The present invention relates to a fermented fruit product having outstanding flavor with a content of $Ca^{2+}$ ion in an amount of 100 ppm or higher on the basis of the total weight of the fermented fruit product, a composition including the same and a preparation method thereof, in which the fermented fruit product has a sugar concentration of 10 brix to 50 brix, the fruit comprises an apple, or the fruit is an apple.

[Background Art]

Calcium is essential for the growth and maintenance of bones and teeth, which functions to help blood coagulation and muscle contraction. Calcium is a major constituent of the hydroxyapatite in the bone, and it acts to strengthen the bones and also functions to store calcium in the body. Further, calcium acts as a critical signal transducer in cells, and the increase or decrease of the calcium concentration mediates the signal transduction process in cells. As another function, calcium is an essential element for controlling the homeostasis of the skin barrier. It is involved in the regeneration process after the skin barrier damage. Calcium administered in a high concentration induces restoration of damaged skin barrier function, an increase in skin hydration and a decrease in inflammation. As such, calcium is a critical and essential nutrient for the growth and composition of the human body, and a sufficient amount of calcium is required to maintain physical health.

However, calcium is a typical essential nutrient having low-intake and is less utilized in the body compared to other nutrients. Most of the calcium-sourced foods, except milk which is a source of calcium, are restricted to anchovy, cheese, laver, soybean, and seaweed and consumed foods are not included, leading to a shortage of calcium intake. Therefore, it is necessary to develop a calcium-containing food to increase calcium intake and to provide an easy and various form so as to increase the intake.

Fruits have excellent flavor and taste so that they are a preferred food group compared with other food groups. Further, fruits are recommended to eat properly because they contain a lot of micronutrients such as vitamins and minerals involved in energy metabolism (Hyesoo Kim et al., J Nutr Health 2014; 47(2): 134-144). Among them, apples are known to be a fruit having familiar taste and flavor and high in preference because of being widely used as food ingredients and having nutritional value and efficacy rich in minerals such as vitamins, calcium, potassium, and polyphenols. However, there is a problem that when calcium and apple are taken together, calcium absorption inhibitor such as cellulose or hemicellulose of an apple interferes with calcium absorption so that it is not suitable.

Further, the fermented apple product converts sugar into a great quantity of organic acid through alcohol fermentation and acetic acid fermentation. At this time, various flavors and tastes are generated depending on the temperature, time, and microbial activity of fermentation. In particular, the fermentation by yeast during the alcohol fermentation process is a critical process to produce unique flavor of the fermented product. Yeast undergoes autolysis according to fermentation, aging and storage state. At this time, enzymes in the yeast decompose carbohydrate, nitrogen component, etc. of cells to break down cell structure. Accordingly, the basic amino acid is discharged to raise the pH and to release the fatty acid having the middle chain ($C_6$ to $C_{12}$). In this case, the substances produced generate esters, aldehydes, and other aromatic substances and flavor components derived from amino acids and single fatty acids can act as odorants. Particularly, the fatty acids having a middle chain ($C_6$ to $C_{12}$) caused due to the autolysis of yeast are oxidized or heated to generate carboxylic acid, which mainly includes hexanoic acid, octanoic acid, and decanoic acid. These substances have an adverse effect on the flavor of the alcohol-fermented product. Further, the amino acids produced by the autolysis of yeast are subjected to heat treatment and the like to generate aldehyde due to the Strecker degradation reaction. Among them, isovaleric acid is produced via a precursor of Leucine, and butanoic acid is produced via a precursor of isoleucine. These substances are one of the substances recognized as odorous when they are contained in large amounts (P. Comuzzo et al. Food chemistry 99, 2006, 217-230). Further, when the yeast is not removed and remains, the growth of acetic acid bacteria may be inhibited to decrease the initial yield of acetic acid fermentation. In the conventional art, acetic acid fermentation was performed mainly after heat sterilization. Thus, there was a problem that heat was applied during the sterilization process, and the odor was generated due to residual yeast that was not killed. Therefore, after the completion of alcohol fermentation, yeast is removed so that the yeast is controlled to prevent autolysis, thereby inhibiting undesirable flavor formation and controlling the flavor.

[Citation List]

[Patent Literature]

[Patent Literature 1]

Korean Patent No.: 10-1321611

[Summary of Invention]

[Technical Problem]

For calcium which is a typical deficient intake-nutrient and is less used in the body, the familiar food sources, particularly apples, having excellent taste and flavor are utilized so as to increase calcium intake and calcium bioavailability, thereby providing calcium in a sufficient amount as a form for convenient intake and increasing calcium bioavailability.

Further, in order to solve the problem that a calcium absorption inhibitor such as cellulose or hemicellulose of an apple interferes with calcium absorption when calcium and apple are taken together, a microbial fermentation technique is applied to fruits to prepare fermented fruit products, thereby converting cellulose, hemicellulose, pectin and the like into low molecular weight components and into organic acids so as to remove the calcium absorption inhibitors and provide calcium in an easy-to-absorb form.

Further, the acetic acid fermentation is performed after conventional heating and sterilizing process during the fruit product fermentation process. In order to overcome the problem of odor caused by residual yeast in which heat is applied and not killed, the membrane filtration, which is non-heat sterilization, is used to eliminate yeast to control the cause of odor. Thus, the present invention provides the fermented fruit product with superior flavor and taste compared with the conventional method in which yeast is removed by heat sterilization.

It contains a great quantity of calcium ions as well as active ingredients of fruits, especially apples. Further, it is easy to use as a food and cosmetic material because of its excellent flavor.

One aspect of the present invention is to provide a fermented fruit product containing a great quantity of calcium with high bioavailability.

One aspect of the present invention is to provide a fermented fruit product containing a great quantity of calcium in ionic form by increasing the solubility of calcium derived from raw calcium material.

One aspect of the present invention is to provide a fresh fermented fruit product with excellent flavor and taste.

One aspect of the present invention is to provide a fresh fermented fruit product with excellent flavor and taste as well as a great quantity of calcium in ionic form.

One aspect of the present invention is to provide a method of preparing a fermented fruit product containing a great quantity of calcium in ionic form by increasing the solubility of calcium derived from raw calcium material.

One aspect of the present invention is to provide a method of preparing a fresh fermented fruit product with excellent flavor and taste.

One aspect of the present invention is to provide a method of preparing a fresh fermented fruit product with excellent flavor and taste as well as a great quantity of calcium in ionic form.

One aspect of the present invention is to provide a food and cosmetic composition including a fermented fruit product containing a great quantity of calcium in ionic form.

One aspect of the present invention is to provide a food and cosmetic composition including a fermented fruit product with excellent flavor or taste.

One aspect of the present invention is to provide a food and cosmetic composition including a fermented fruit product with excellent flavor and taste as well as a great quantity of calcium in ionic form.

[Solution to Problem]

One aspect of the present invention provides a fermented fruit product containing organic acid, in which $Ca^{2+}$ ions are contained in an amount of 100 ppm or higher on the basis of the total weight of the fermented fruit product.

One aspect of the present invention provides the fermented fruit product of a fruit concentrate having a sugar content of 10 brix to 50 brix.

One aspect of the present invention provides a fermented fruit product, in which the fruit comprises an apple.

One aspect of the present invention provides a fermented fruit product, in which the fruit is an apple.

One aspect of the present invention provides a fermented fruit product, in which $Ca^{2+}$ ions are contained in an amount of 1,000 ppm or higher on the basis of the total weight of the fermented fruit product.

One aspect of the present invention provides a fermented fruit product, in which $Ca^{2+}$ ions are contained in an amount of 5,000 ppm or higher on the basis of the total weight of the fermented fruit product.

One aspect of the present invention provides the fermented fruit product which is by an alcohol fermentation and an acetic acid fermention.

One aspect of the present invention provides a fermented fruit product which is a fermented product by *Saccharomyces* genus yeast and *Acetobacter* genus acetic acid bacteria.

One aspect of the present invention provides a fermented fruit product, in which the organic acid is contained in an amount of 10,000 ppm or higher on the basis of the total weight of the fermented fruit product and a free sugar is contained in an amount of 25,000 ppm or lower on the basis of the total weight of the fermented fruit product.

Another aspect of the present invention provides a composition comprising the fermented fruit product as described above, wherein the composition is a food or a cosmetic composition.

Still another aspect of the present invention provides a method of preparing the fermented fruit product, the method including: alcohol-fermenting a raw fruit material with yeast; and acetic acid-fermenting the alcohol-fermented product with acetic acid bacteria to obtain a fermented fruit product, in which a raw calcium material is added at a concentration of 0.05% by weight (w/v) or more on the basis of the total volume of the alcohol fermented product during the acetic acid fermentation, and in which, during the acetic acid fermentation, the raw calcium material is added at the time when a content of the organic acid reaches 10,000 ppm or higher in the fermented product.

Still another aspect of the present invention provides a method of preparing a fermented fruit product, wherein the method further comprises removing the yeast by non-heat sterilization after completion of the alcohol fermentation.

Still another aspect of the present invention provides a method of preparing a fermented fruit product, in which the non-heat sterilization comprises one or more selected from the group consisting of ultraviolet sterilization, membrane filtration sterilization, high voltage pulsed magnetic field sterilization, ultrahigh pressure sterilization, and ultrasonic sterilization.

Still another aspect of the present invention provides a method of preparing a fermented fruit product, in which the non-heat sterilization is membrane filtration sterilization.

Still another aspect of the present invention provides a method of preparing a fermented fruit product, in which, during the membrane filtration sterilization, the pore size of the membrane may be 5 um or less, 1 um or less, 0.65 um or less, 0.45 um or less, 0.2 um or less, or 0.1 um or less.

Still another aspect of the present invention provides a method of preparing a fermented fruit product, in which removing the yeast is carried out at a temperature of 10°C to 40°C.

Still another aspect of the present invention provides a method of preparing a fermented fruit product, in which, during the acetic acid fermentation, the raw calcium material is added at the time when a content of the organic acid in the fermented product is 10,000 ppm to 100,000 ppm.

Still another aspect of the present invention provides a method of preparation a fermented fruit product, the method further comprising, during the acetic acid fermentation, aging after the addition of the raw calcium material.

Still another aspect of the present invention provides a method of preparation a fermented fruit product, wherein the method further comprises lactic acid-fermenting using the lactic acid bacteria before the alcohol fermentation.

Still another aspect of the present invention provides a method, in which the yeast is *Saccharomyces* genus, the acetic acid bacteria is *Acetobacter* genus, and the lactic acid bacteria is *Lactobacillus* genus.

Still another aspect of the present invention provides a method, in which the *Saccharomyces* genus is *Saccharomyces cerevisiae,* the *Acetobacter* genus is *Acetobacter aceti,* and the *Lactobacillus* genus is *Lactobacillus casei.*

In still another aspect of the present invention, the raw calcium material may be a raw material comprising calcium, and more particularly, but is not limited to, calcium-class which can be used as a food additive.

Still another aspect of the present invention provides a method, in which the raw calcium material comprises one or more selected from the group consisting of calcium citrate, calcium gluconate, calcium 5-ribonucleotide, calcium hydroxide, calcium stearyl lactate, calcium chloride, calcium disodium EDTA, calcium lactate, Calcium hydroxyapatite, Calcium hydrogen phosphate, Calcium dihydrogen phosphate, carboxymethylcellulose calcium, calcium carbonate, calcium pantothenate, calcium propionate, calcium sulfate, calcium glycerophosphate, calcium oxide, calcium ascorbate, calcium alginate, calcium acetate, calcium benzoate, calcium stearate, calcium sorbate, calcium ferrocyanide, and calcium caseinate.

Still another aspect of the present invention provides a method, in which the calcium carbonate comprises one or more selected from the group consisting of eggshell calcium, shell calcium, seaweed calcium, and pearl calcium.

[Advantageous Effects of Invention]

The fermented product according to one aspect of the present invention provides a fermented fruit product with improved flavor and taste, which facilitates calcium intake in accordance with consumer preferences.

The fermented product according to one aspect of the present invention has an excellent bioavailability of calcium because a great quantity of organic acid contained in the fermented product ionizes the calcium.

In the fermented fruit product containing a great quantity of calcium ion according to the present invention, when 700 mL of the fermented fruit product containing 1,000 ppm of calcium ion content is consumed, it results in an effect of taking the content satisfying 700 mg of the recommended daily intake of Korean female aged 19 to 49.

In the fermented fruit product containing a great quantity of calcium ion according to the present invention, when 140 mL of the fermented fruit product containing 5,000 ppm of calcium ion content is consumed, it results in an effect of taking the content satisfying 700 mg of the recommended daily intake of Korean female aged 19 to 49.

In the fermented fruit product containing a great quantity of calcium ion according to the present invention, when 70 mL of the fermented fruit product containing 10,000 ppm of calcium ion content is consumed, it results in an effect of taking the content satisfying 700 mg of the recommended daily intake of Korean female aged 19 to 49.

The fermented product according to one aspect of the present invention is excellent in fatigue recovery because it contains a great quantity of organic acid.

The fermented product according to one aspect of the present invention has an excellent effect of promoting internal or skin absorption of calcium component included in the fermented product.

The fermented product according to one aspect of the present invention has a high content of $Ca^{2+}$ ions.

The fermented product according to one aspect of the present invention or the composition according to the other aspect of the present invention has an excellent effect in helping blood coagulation.

The fermented product according to one aspect of the present invention or the composition according to the other aspect of the present invention has an excellent effect in helping muscle contraction.

The fermented product according to one aspect of the present invention or the composition according to the other aspect of the present invention has an excellent effect in controlling skin barrier homeostasis.

The fermented product according to one aspect of the present invention or the composition according to the other aspect of the present invention has an excellent effect in recovering a skin barrier function.

The fermented product according to one aspect of the present invention or the composition according to the other aspect of the present invention has an excellent effect in increasing skin hydration.

The fermented product according to one aspect of the present invention or the composition according to the other aspect of the present invention has an excellent effect in reducing inflammation.

The fermented product according to one aspect of the present invention or the composition according to the other aspect of the present invention has an excellent effect in the absorption of calcium in the skin.

The fermented product according to one aspect of the present invention or the composition according to the other aspect of the present invention has an excellent effect in the absorption of calcium in the body.

A method of preparing a composition according to still another aspect of the present invention provides a method of preparing a fermented product having an excellent effect in enhancing internal or skin absorption of calcium component included in the fermented product.

[Brief Description of Drawings]

FIG. 1 is a flowchart of the method of preparing a fermented apple product according to Examples 2 and 3 of the present invention.

FIG. 2 is a graph illustrating the calcium ion content of Example 2 according to the experimental examples 1-2 of the present invention.

FIG. 3 is a graph illustrating the alcohol content during alcohol fermentation according to experimental example 2 of the present invention.

FIG. 4 is a graph illustrating changes in total acidity during acetic acid fermentation according to experimental example 2 of the present invention.

FIGS. 5 to 7 are graphs illustrating experimental results of the flavor pattern of the experimental example 3 of the present invention, in which FIG. 5 is for comparative example 2, FIG. 6 is for Example 1, and FIG. 7 is for Example 3.

FIG. 8 is a view illustrating the results of the flavor sensory test of experimental example 4.

FIG. 9 is a view illustrating the result of analyzing free sugar according to experimental example 5.

[Description of Embodiments]

Hereinafter, the present invention is described in detail.

One aspect of the present invention provides a fermented fruit product containing organic acid, in which $Ca^{2+}$ ions are contained in an amount of 100 ppm or higher on the basis of the total weight of the fermented fruit product.

One aspect of the present invention provides a fermented fruit product containing a fermented product of fruit concentrate having a sugar content of 10 brix to 50 brix. In particular, the sugar content of fruit concentrate may be 10 brix or more, 15 brix or more, or 20 brix or more, and may be 50 brix or less, 45 brix or less, or 40 brix or less.

One aspect of the present invention provides a fermented fruit product, in which the fruit comprises an apple.

One aspect of the present invention provides a fermented fruit product, in which the fruit is an apple.

One aspect of the present invention provides a fermented fruit product, in which $Ca^{2+}$ ions are contained in an amount of 1,000 ppm or higher on the basis of the total weight of the fermented fruit product.

One aspect of the present invention provides a fermented fruit product, in which $Ca^{2+}$ ions are contained in an amount of 5,000 ppm or higher on the basis of the total weight of the fermented fruit product.

One aspect of the present invention provides a composition, in which $Ca^{2+}$ ions are contained in an amount of 1,000 ppm to 40,000 ppm on the basis of the total weight of the fermented fruit product.

In particular, a content of the $Ca^{2+}$ ions may be 100 ppm or higher, 500 ppm or higher, 1,000 ppm or higher, 5,000 ppm or higher, 7,000 ppm or higher, 8,000 ppm or higher, 9,000 ppm or higher, 10,000 ppm or higher, 11,000 ppm or higher, 12,000 ppm or higher, 15,000 ppm or higher, 20,000 ppm or higher or 30,000 ppm or higher on the basis of the total weight of the fermented fruit product. Further, the content of $Ca^{2+}$ ion may be 40,000 ppm or lower, 30,000 ppm or lower, 20,000 ppm or lower, 17,000 ppm or lower, 14,000 ppm or lower, 13,000 ppm or lower, 12,000 ppm or lower, or 10,000 ppm or lower on the basis of the total weight of the fermented fruit product.

One aspect of the present invention includes a fermented fruit product containing organic calcium. The organic calcium may be present in the easy-to-absorb form of a $Ca^{2+}$ ion into the body or skin by the organic acid component of the fermented fruit product. Calcium in the form of general inorganic calcium is not present enough in the form of $Ca^{2+}$ ion, thereby decreasing the body or skin absorption rate. Thus, the fermented product has sufficient amount of $Ca^{2+}$ ions so that it is easy to use.

In this specification, the term 'body' refers to the interior of the body of a subject to which the composition is administered, and the term 'skin' refers to the outer cover of the body of a subject to which the composition is administered.

In the case of apple vinegar made from apple as the main ingredient, the sugar is converted into 'alpha hydroxy acid' type organic acid (fruit acid, glycolic acid, citric acid, malic acid, tartaric acid, etc.) due to the fermentation process of yeast, lactic acid bacteria, and acetic acid bacteria. The organic acids caused during fermentation provide conditions that are easy to dissolve calcium, thereby increasing the bioavailability of calcium. Thus, one aspect of the present invention is to provide a fermented product that can effectively dissolve the raw calcium material to improve the absorption of the organic calcium in the body.

In one aspect of the present invention, the raw apple material may be, but is not limited to, apple, apple juice, and apple concentrate.

The apple (*Malus* domestica) may be included in one or more of the species: early variety (Sunhong, Tsugaru, Sansa, Seogwang), medium variety (Hongo, Gamhong, Yangkwang, Chuwang, Hongwol, Goul, Hirosaki, Jonagold, Hongwok, Yangkwang), and late variety (Fuji, Hawhong, royal fuji), but the present invention is not limited thereto.

One aspect of the present invention provides the fermented fruit product which is an alcohol and acetic acid fermented product.

One aspect of the present invention provides a fermented fruit product which is a fermented product by *Saccharomyces* genus yeast and *Acetobacter* genus acetic acid bacteria.

One aspect of the present invention provides a fermented fruit product, in which the organic acid is contained in an amount of 10,000 ppm or higher on the basis of the total weight of the fermented fruit product and a free sugar is contained in an amount of 25,000 ppm or lower on the basis of the total weight of the fermented fruit product.

In one aspect of the present invention, a content of organic acid may be 10,000 ppm to 170,000 ppm on the basis of the total weight of the fermented fruit product. In particular, the content of organic acid may be 10,000 ppm or higher, 15,000 ppm or higher, 20,000 ppm or higher, 25,000 ppm or higher, 30,000 ppm or higher, 35,000 ppm or higher, 40,000 ppm or higher, 45,000 ppm or higher, 48,000 ppm or higher, 50,000 ppm or higher, 55,000 ppm or higher, 60,000 ppm or higher, or 65,000 ppm or higher on the basis of the total weight of the fermented fruit product. Further, the content of organic acid may be 170,000 ppm or lower, 150,000 ppm or lower, 100,000 ppm or lower, 90,000 ppm or lower, 80,000 ppm or lower, 70,000 ppm or lower, or 65,000 ppm on the basis of the total weight of the fermented fruit product. When the content of organic acid meets the range as described above, the sugar component contained in the raw material is sufficiently converted into the organic acid through the fermentation process, so that the calcium salt-type calcium is dissociated to increase the content of $Ca^{2+}$ ion which may be easily absorbed.

Further, in the fermented fruit product, the content of free sugar may be 25,000 ppm or lower, 20,000 ppm or lower, 5,000 ppm or lower, 3,500 ppm or lower, or 3,000 ppm or lower on the basis of the total weight of the fermented fruit product. When the content of free sugar meets the range as described above, the sugar component contained in the raw material is sufficiently converted into the organic acid through the fermentation process, so that the calcium salt-type calcium is dissociated to increase the content of $Ca^{2+}$ ion which may be easily absorbed.

Another aspect of the present invention provides a food or cosmetic composition including the fermented fruit product.

In another aspect of the present invention, the food or cosmetic composition may further include additives necessary within the range for the purpose of each food or cosmetic composition.

Still another aspect of the present invention provides a method of preparing a fermented fruit product, the method including: alcohol-fermenting a raw fruit material with yeast; and acetic acid-fermenting the alcohol-fermented product with acetic acid bacteria to obtain a fermented fruit product, in which a raw calcium material is added at a concentration of 0.05% by weight (w/v) or more on the basis of the total volume of the alcohol fermented product during the acetic acid fermentation, and in which, during the acetic acid fermentation, the raw calcium material is added at the time when a content of the organic acid reaches 10,000 ppm or higher in the fermented product.

In the method, alcohol-fermenting a raw fruit material with the yeast may include increasing the alcohol content to a high concentration by gradually adding a raw fruit material. Thus, still another aspect of the present invention provides a method of preparing a fermented fruit product in which alcohol-fermenting includes fermentation in which the addition is initiated at the time when a alcohol content of the alcohol-fermented product reaches 12% or more, then a process of adding 3% by weight to 7% by weight of the raw fruit material on the basis of the total weight of the alcohol-fermented product is repeated two times or more, and thus the fermentation is maintained until the final alcohol content of the alcohol-fermented product reaches 15% or more. The further addition count may be 3 times to 5 times. Further, the addition time may be the time when the alcohol content of the alcohol-fermented product is 12% or more, 12.5% or more, 13% or more, 13.5% or more, 14% or more, 14.5% or more, or 15% or more. Further, the final alcohol content may be 15% or more, 15.5% or more or 16% or more. The further added raw fruit material may be 3% by weight or more, 3.5% by weight or more, 4% by weight or more, 4.5% by weight or more, 5% by weight or more, or 5.5% by weight or more on the basis of the total weight of the alcohol-fermented product. Further, it may be 7% by weight or less, 6.5% by weight or less, 6.0% by weight or less, 5.5% by weight or less, or 5% by weight or less on the basis of the total weight of the alcohol-fermented product.

In the method, acetic acid-fermenting the alcohol-fermented product with an acetic acid bacteria may include preparing an acetic acid-fermented product with high acidity by gradually adding the alcohol-fermented product. Thus, still another aspect of the present invention provides a method of preparing a fermented fruit product in which acetic acid-fermenting includes fermentation in which the addition is initiated at the time when the acidity of the acetic acid-fermented product reaches 5% or more, then a process of adding 15% by weight to 20% by weight of the alcohol-fermented product on the basis of the total weight of the acetic acid-fermented product is repeated two times or more, and thus the fermentation is maintained until the final acidity of the acetic acid-fermented product reaches 9% or more. The further addition count may be 3 times to 5 times. Further, the addition time may be the time when the acidity of the acetic acid-fermented product is 5% or more, 5.5% or more, 6% or more, 6.5% or more, 7% or more, 7.5% or more, or 8% or more. Further, the final acidity of the acetic acid-fermented product may be 9% or more, 9.5% or more or 10% or more. The further added alcohol-fermented product may be 15% by weight or more, 15.5% by weight or more, 16% by weight or more, 16.5% by weight or more, 17% by weight or more, or 17.5% by weight or more on the basis of the total weight of the acetic acid-fermented product. Further, it may be 20% by weight or less, 19.5% by weight or less, 19% by weight or less, 18.5% by weight or less, or 18% by weight or less on the basis of the total weight of the acetic acid-fermented product.

A fermentation process technology that converts to a great quantity of organic acid is needed so as to dissolve calcium in an easy-to-absorb form. In order to prepare vinegar containing a high concentration of acetic acid having a concentration of 10% or more of organic acid, a high content of alcohol is required. In order to prepare a high content of alcohol, a high concentration of glycogen is required. However, a high concentration of glycogen may act as a factor of inhibiting the growth of yeast. Futher, a high concentration of alcohol conent may act as a factor of inhibiting the growth of acetic acid bacteria. Thus, since it is hard to prepare an alcohol-fermented product with a high concentration, the acetic acid fermentation is typically proceeded by addition of alcohol when acetic acid-fermented product with high acidity is produced. However, the present invention is to provide a fermented fruit product containing a great quantity of organic acid by preparing a highly concentrated alcohol-fermented product and a highly concentrated acetic acid-fermented product through a stepwise addition process of high-concentration raw material. Accordingly, the present invention is to provide a fermented fruit product in which calcium may be dissolved in a great quantity and calcium may be provided in a great quantity of ionized form of calcium with high bioavailability.

Still another aspect of the present invention provides a method of preparing a fermented fruit product, wherein the method further comprises removing the yeast by non-heat sterilization after completion of the alcohol fermentation.

Still another aspect of the present invention provides a method of preparing a fermented fruit product, in which the non-heat sterilization comprises one or more selected from the group consisting of ultraviolet sterilization, membrane filtration sterilization, high voltage pulsed magnetic field sterilization, ultrahigh pressure sterilization, and ultrasonic sterilization. The non-heat sterilization of removing yeast results in the free-odor and complete removal of the residual yeast, thereby improving the flavor and taste of the final fermented product.

Still another aspect of the present invention provides a method of preparing a fermented fruit product, in which the non-heat sterilization is membrane filtration sterilization.

Still another aspect of the present invention provides a method of preparing a fermented fruit product, in which, during the membrane filtration sterilization, the pore size of the membrane may be 5 um or less, 1 um or less, 0.65 um or less, 0.45 um or less, 0.2 um or less, or 0.1 um or less.

Still another aspect of the present invention provides a method of preparing a fermented fruit product, in which removing the yeast is carried out at a temperature of 10°C to 40°C. In particular, removing the yeast may be carried out at a temperature of 10°C or more, 13°C or more, 15°C or more, 17°C or more, 20°C or more, 25°C or more, 28°C or more, or 30°C or more. Further, removing the yeast may be carried out at a temperature of 40°C or less, 38°C or less, 36°C or less, 35°C or less, 34°C or less, 33°C or less, 30°C or less, or 28°C or less. Preferably, removing the yeast may be carried out at a temperature of 15°C to 35°C.

Still another aspect of the present invention provides a method of preparing a fermented fruit product, in which, during the acetic acid fermentation, the raw calcium material is added at the time when the content of the organic acid in the fermented product is 10,000 ppm or higher. In particular, during the acetic acid fermentation, the raw calcium material may be added at the time when the content of the organic acid in the fermented product is 10,000 ppm or higher, 15,000 ppm or higher, 20,000 ppm or higher, 25,000 ppm or higher, 30,000 ppm or higher, 35,000 ppm or higher, 40,000 ppm or higher, 50,000 ppm or higher, 60,000 ppm or higher, 70,000 ppm or higher, 80,000 ppm or higher, 90,000 ppm or higher, or 100,000 ppm or higher. Further, it may be added at the time when the content of the organic acid in the fermented product is 150,000 ppm or lower, 140,000 ppm or lower, 130,000 ppm or lower, 110,000 ppm or lower, 100,000 ppm or lower, 95,000 ppm or lower, 90,000 ppm or lower, 80,000 ppm or lower, 50,000 ppm or lower, 30,000 ppm or lower, or 25,000 ppm or lower.

Still another aspect of the present invention provides a method of preparing a fermented fruit product, in which, during the acetic acid fermentation, the raw calcium material is added at the time when the content of the organic acid in the fermented product is 70,000 ppm to 100,000 ppm.

Still another aspect of the present invention provides a method of preparing a fermented fruit product, the method further including, during the acetic acid fermentation, aging and fermenting after the addition of the raw calcium material.

In another aspect of the present invention, the raw fruit materail may be a fruit, fruit juice, or fruit concentrate.

In still another aspect of the present invention, the raw calcium material may be a raw material including calcium, and more particularly, but is not limited to, calcium-class which can be used as a food additive.

Still another aspect of the present invention provides a method, in which the raw calcium material comprises one or more selected from the group consisting of calcium citrate, calcium gluconate, calcium 5-ribonucleotide, calcium hydroxide, calcium stearyl lactate, calcium chloride, calcium disodium ETDA, calcium lactate, Calcium hydroxyapatite, Calcium hydrogen phosphate, Calcium dihydrogen phosphate, carboxymethylcellulose calcium, calcium carbonate, calcium pantothenate, calcium propionate, calcium sulfate, calcium glycerophosphate, calcium oxide, calcium ascorbate, calcium alginate, calcium acetate, calcium benzoate, calcium stearate, calcium sorbate, calcium ferrocyanide, and calcium caseinate.

Still another aspect of the present invention provides a method, in which the calcium carbonate comprises one or more selected from the group consisting of eggshell calcium, shell calcium, seaweed calcium, and pearl calcium.

In still another aspect of the present invention, the raw calcium material may be shell calcium, pearl calcium, calcium lactate, calcium hydroxide, calcium carbonate, or seaweed calcium as a raw material for food or cosmetics depending on its purpose, but the range thereof is not limited thereto. The raw calcium material may be added in a content of 0.05% to 20% (w/v) for the preparation depending on its purpose.

In still another aspect of the present invention, the fermented fruit product may be one prepared by using apples (including pericarp) as a raw material, being subjected to an enzymatic reaction to decompose components such as cellulose, hemicellulose and pectin, which are insoluble or soluble fibrin, if necessary, and proceeding the alcohol fermentation and acetic acid fermentation (if necessary, the lactic acid-fermentation is performed).

In still another aspect of the present invention, the alcohol fermentation may be performed at a temperature of 15°C to 35°C by inoculating 0.1% to 1.0% (v/v) of yeast into the raw apple material. The juice or concentrate may be titrated to a brix of 20 to 40. The alcohol content of the fermented fruit product obtained after the completion of alcohol fermentation may be 10% to 20%. In order to obtain a high alcohol concentration, an raw apple material may be added in 3 steps to 4 steps to obtain a fruit wine with a high concentration. After the completion of alcohol fermentation, the removal of yeast (and lactic acid bacteria) may be performed by the sterilization through membrane filtration sterilization method, which is one of the non-heat sterilization methods.

In still another aspect of the present invention, the acetic acid fermentation may be performed at a temperature of 25°C to 35°C by inoculating 0.1% to 1.0% (v/v) of acetic acid bacterial culture medium into the fermented fruit product. At this time, in order to obtain an acetic acid-fermented product containing a high concentration of organic acid, the fruit wine containing a high-concentration alcohol may be added in 4 steps to 5 steps to carry out acetic acid fermentation and aging and fermentation, thereby preparing the fermented fruit product. Here, the total acidity of the fermented fruit product prepared may be 4.5% to 12.0%.

Still another aspect of the present invention provides a method of preparing a fermented fruit product, wherein the method further comprises lactic acid-fermenting using the lactic acid bacteria before the alcohol fermentation.

In another aspect of the present invention, the lactic acid-fermentation may be performed depending on its purpose, and is carried out at a temperature of 25°C to 35°C by inoculating 0.1% to 1.0% (v/v) of lactic acid bacteria thereinto. The alcohol content of the ethanol-fermented product obtained after completion of the lactic acid-fermentation and alcohol fermentation may be 10% to 20%.

Still another aspect of the present invention provides a method, in which the yeast *is Saccharomyces* genus, the acetic acid bacteria is *Acetobacter* genus, and the lactic acid bacteria is *Lactobacillus* genus.

Still another aspect of the present invention provides a method, in which the *Saccharomyces* genus is *Saccharomyces cerevisiae,* the *Acetobacter* genus is *Acetobacter aceti,* and the *Lactobacillus* genus is *Lactobacillus casei.*

In still another aspect of the present invention, the *Saccharomyces cerevisiae* may be a microorganism owned by Sempio foods co., Ltd, which is a microorganism deposited with the microorganism deposit number KCTC 12749BP on January 22, 2015, at the Korean Collection for Type Cultures of the Korea Research Institute of Bioscience and Biotechnology.

In still another aspect of the present invention, the *Acetobacter aceti* may be obtained by isolating a bacterium having excellent growth and acetic acid production from vinegar into which rice wine is naturally fermented. It may be obtained by performing the shaking-culture using YPGD (W/0.5% acetic acid, 6% ethanol) medium (YPGD medium composition: 1% yeast extract, 1% peptone, 2% glycerol, 0.5% glucose, 0.5% acetic acid, and 6% ethanol) under an aerobic condition at a temperature of 25°C to 33°C.

In still another aspect of the present invention, the *Lactobacillus casei* may be obtained by isolating a strain having excellent growth and flavor from rice wine. It may be obtained by performing the static-culture using MRS broth medium (Difco™ Lactobacilli MRS broth: 10 g proteose peptone No. 3, 10 g beef extract, 5g yeast extract, 20 g dextrose, 1 g polysorbate 80, 2 g ammonium citrate, 5 g sodium acetate, 0.1 g magnesium sulfate, 0.05 g manganese sulfate, 2 g dipotassium phosphate per 1L) under an anaerobic condition at a temperature of 28°C to 37°C.

[Embodiments]

Hereinafter, the present invention is described in more detail. It is apparent to those skilled in the art that these Examples are merely illustrative of the present invention and that the scope of the present invention is not to be construed as limited by these Examples.

[Comparative example 1] Apple concentrate

A commercially available apple concentrate (raw apple material) was used as a comparative example 1. The organic acid and organic sugar content of the apple concentrate of Comparative example 1 was confirmed in experimental example 5 as described below.

[Comparative example 2] Fermented apple product with heat sterilization

The apple concentrate of Comparative example 1 was fermented with microorganisms to prepare a fermented apple product.

After the raw apple material was mixed and diluted with purified water at a volume ratio of 1:1, the yeast (yeast culture solution: *Saccharomyces cerevisiae* of Accession number KCTC 12749BP) was added thereto in 0.1% to 1.0% (v/v) on the basis of the total weight thereof, and the alcohol fermentation was carried out at 15°C to 30°C to obtain apple wine. The obtained apple wine was heated so that the material temperature reached 60°C to 70°C to be sterilized. The apple wine was mixed with purified water at a volume ratio of 1:1. Then, 0.1% to 1.0% (v/v) of acetic acid culture medium (a culture medium containing *Acetobacter aceti*) was inoculated into the mixture on the basis of the total liquid amount thereof. Then Acetic acid fermentation and aging were performed at 25°C to 30°C, thereby preparing a fermented apple product.

[Comparative example 3] Purified water added with seaweed calcium

4% (w/v) of commercially available seaweed calcium was added to purified water, and they were stirred.

[Comparative example 4] Fermented apple product added with raw calcium material during alcohol fermentation

While the apple concentrate of Comparative example 1 was fermented with a microorganism, the raw calcium material was added thereto during the alcohol fermentation, thereby preparing the fermented apple product. After the raw apple material was mixed and diluted with purified water at a volume ratio of 1:1, the yeast (yeast culture solution: *Saccharomyces cerevisiae* of Accession number KCTC 12749BP) was added thereto in 0.1% to 1.0% (v/v) on the basis of the total weight thereof, and 1.0% (w/v) calcium hydroxide was added thereto. Then, the alcohol fermentation was carried out at 15°C to 30°C to obtain apple wine. The obtained apple wine was heated so that the material temperature reached 60°C to 70°C to be sterilized. The apple wine was mixed with purified water at a volume ratio of 1:1. Then, 0.1% to 1.0% (v/v) of acetic acid culture medium (a culture medium containing *Acetobacter aceti*) was inoculated into the mixture on the basis of the total liquid amount thereof. Then, Acetic acid fermentation and aging were performed at 25°C to 30°C, thereby preparing a fermented apple product.

[Comparative example 5] Fermented apple product added with raw calcium material during initial acetic acid fermentation

While the apple concentrate of Comparative example 1 was fermented with microorganisms, the raw calcium material was added thereto during the initial acetic acid fermentation, thereby preparing the fermented apple product. After the raw apple material was mixed and diluted with purified water at a volume ratio of 1:1, the yeast (yeast culture solution: *Saccharomyces cerevisiae* of Accession number KCTC 12749BP) was added thereto in 0.1% to 1.0% (v/v) on the basis of the total weight thereof. Then, the alcohol fermentation was carried out at 15°C to 30°C to obtain apple wine. The obtained apple wine was heated so that the material temperature reached 60°C to 70°C to be sterilized. The apple wine was mixed with purified water at a volume ratio of 1:1. Then, 0.1% to 1.0% (v/v) of acetic acid culture medium (a culture medium containing Acetobacter aceti) was inoculated into the mixture on the basis of the total liquid amount thereof. 1.0% (w/v) calcium hydroxide was added thereto. Acetic acid fermentation and aging were performed at 20°C to 30°C, thereby preparing a fermented apple product.

[Example 1] Non-heat sterilized fermented apple product

While the apple concentrate of Comparative example 1 was fermented with microorganisms, the fermented apple product was prepared by removing yeast through non-heat sterilization it is different from Comparative example 2. After the raw apple material was mixed and diluted with purified water at a volume ratio of 1:1, the yeast (yeast culture solution: *Saccharomyces cerevisiae* of Accession number KCTC 12749BP) was added thereto in 0.1% to 1.0% (v/v) on the basis of the total weight thereof. Then, the alcohol fermentation was carried out at 15°C to 30°C, and then the yeast was removed to obtain apple wine. In order to obtain a high concentration of alcohol, the apple concentrate was added in 3 steps to 4 steps in 5% parts by weight on the basis of the total liquid amount to obtain a high concentration of apple wine. The removal of yeast was carried out by membrane filtration sterilization, which is one of the non-heat sterilization processes, in which a filtration membrane having a size of 0.45 um was used. The apple wine was mixed with purified water at a volume ratio of 1:1. Then, 0.1% to 1.0% (v/v) of acetic acid culture medium (a culture medium containing Acetobacter aceti) was inoculated into the mixture on the basis of the total liquid amount thereof. Then, acetic acid fermentation was performed at 25°C to 30°C. Further, the apple wine containing a high concentration of alcohol was added thereto in 4 steps to 5 steps in 17% parts by weight on the basis of the total liquid amount in order to obtain acetic acid-fermented product containing high concentration of organic acid. Then, acetic acid fermentation and aging were performed to prepare a fermented apple product.

[Example 2] Fermented apple product containing a great quantity of calcium ion (derived from calcium hydroxide) according to the amount of raw calcium material added

The same manner of Example 1 was used except that 0.1%, 1.0%, 2.0%, 3.0%, 4.0%, 5.0%, 10.0%, and 30.0% calcium hydroxide were added thereto during the acetic acid fermentation, and acetic acid fermentation and aging were performed to prepare a fermented apple product having a great quantity of calcium ions. At this time, seaweed calcium was added at the time when the content of organic acid reached 90,000 ppm after the acetic acid fermentation proceeded, rather than the beginning of acetic acid fermentation.

[Example 3] Fermented apple product containing a great quantity of calcium ion (derived from calcium hydroxide)

The same manner of Example 1 was used except that 1% (w/v) calcium hydroxide was added thereto during the acetic acid fermentation, and acetic acid fermentation and aging were performed to prepare a fermented apple product having a great quantity of calcium ions. At this time, calcium hydroxide was added at the time when the content of organic acid reached 90,000 ppm after the acetic acid fermentation proceeded, rather than the beginning of acetic acid fermentation.

[Example 4] Fermented apple product containing a great quantity of calcium ion (derived from seaweed calcium)

The same manner of Example 1 was used except that 4% (w/v) seaweed calcium was added thereto during the acetic acid fermentation, and acetic acid fermentation and aging were performed to prepare a fermented apple product having a great quantity of calcium ions. At this time, seaweed calcium was added at the time when the content of organic acid reached 90,000 ppm after the acetic acid fermentation proceeded, rather than the beginning of acetic acid fermentation.

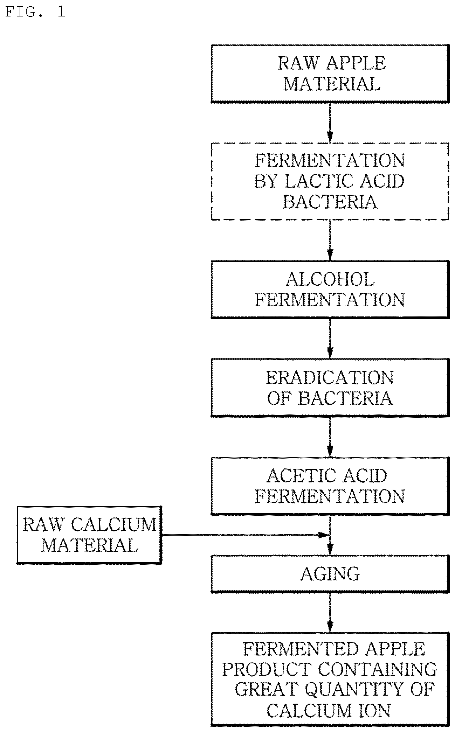

The preparation process of fermented apple products according to Examples 2 to 4 is illustrated in FIG. 1.

Comparative examples and Examples as prepared above are used in the following experiments.

[Experimental example 1-1] Analysis of calcium ion content

The calcium contents of Comparative example 3 and Examples 1 and 4 were compared. The method of measuring calcium was performed as follows.

After each sample was filtered, the content of ionized calcium was measured using a Cedex Bio calcium measuring instrument. The calcium ion contained in the sample formed a complex with 5-nitro-5'-methyl-BAPTA contained in the indicator. The complex is reacted with the EDTA-added indicator. Then, the calcium ion formed a strong chelator with EDTA. As a principle in which the absorbance changes proportionally with the calcium concentration, the device itself measured the calcium ion content in the sample. The results of comparing calcium contents are shown in Table 1 below.

[Table 1]

| Item | Comparative example 3 (purified water + 4% seaweed) | Example 1 (fermented apple product) | Example 4 (fermented apple product + 4% seaweed calcium) |
|---|---|---|---|
| Calcium content (ppm) | 19.6 | 92 | 11,272 |

The results of the experiment indicated that the calcium content of Example 4 in which the fermentation was performed by adding the raw calcium material (seaweed calcium) was about 575 times or more as compared with that of Comparative example 3, and the calcium content of Example 2 was increased about 100 times or more as compared with that of Example 1.

[Experimental example 1-2] Analysis of calcium ion content of Example 2

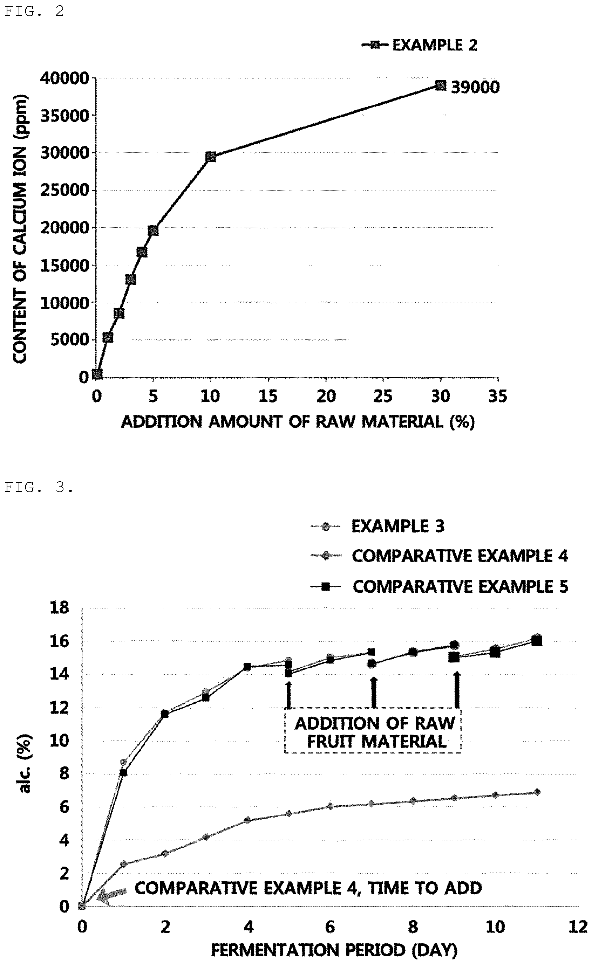

The calcium contents of the fermented apple product according to the addition amount of the raw calcium material in Example 2 were compared by the same manner used in Experimental example 1-1. The results of measuring calcium ion content according to the amount of raw calcium material added are illustrated in FIG. 2.

The results of the experiment indicated that 30% of the calcium hydroxide raw material was added to be dissolved and ionized up to 39,000 ppm.

[Experimental example 2] Measurement of calcium ion content, alcohol content, and total acidity according to the time of adding raw calcium material

The calcium ion contents of Comparative examples 4 and 5 and Example 3 were compared to confirm whether the raw calcium material was lost according to the timing of adding the raw calcium material. The alcohol content and total acidity were measured so as to adjust the right timing of adding the raw material.

(1) Calcium was measured in the same manner as in Experimental example 1.

(2) Alcohol content was taken up to the scale of a 100 ml messflask, which was assayed at 15°C. This was transferred to a 300 to 500 mL flask, and then the messflask was washed twice with about 15 mL of water. The washed solution was added into the flask. It was connected to a cooler. The solution was distilled using the messflask as a receiving container. When the distillate reached 70 mL (the time required was about 20 minutes), the distillation was stopped. Water was added to the scale of the messflask at 15°C. Then, the distillate was shaken well and transferred to a cylinder. Then, the alcohol contents were measured using auto density measurer (Density meter, Anton paar, DMA4500M), a device for analyzing ethanol.

(3) The total acidity was measured in which 10 mL of sample was collected, the distilled water was added thereto to be 100 mL, and 20 mL of the liquid was titrated with 0.1 N sodium hydroxide solution using phenolphthalein solution as an indicator (0.1 N sodium hydroxide solution = 0.006 g $CH_3COOH$).

$$Total\ acidity\ (w/v\%,\ as\ acetic\ acid) = S \times ((f \times$$

$$0.006) \div collected\ sample\ weight\ (g)) \times 100$$

S: consumption of 0.1 N sodium hydroxide solution (mL)
f: titer of 0.1 N sodium hydroxide solution

The consumption of sodium hydroxide was measured, and the total acidity was calculated using this equation.
The results of the experiments are as follows.
(1) Calcium content is as shown in Table 2 below.

[Table 2]

|  | Theoretical value | Comparative example 4 | Comparative example 5 | Example 3 |
|---|---|---|---|---|
| Calcium ion content (ppm) | 5400 | 1,724 | 4608 | 5352 |
| Raw material solubility (%) | - | 32 | 86 | 99 |
| (Calculation of theoretical values: *See* the conversion coefficient table of inorganic raw material (calcium) specified in the Korean Health Functional Foods Act and Food Additives Act) | | | | |

$$Raw\ mateiral\ solubility\ (\%) = calcium\ ion\ content\ /$$

$$theoretical\ value\ content \times 100$$

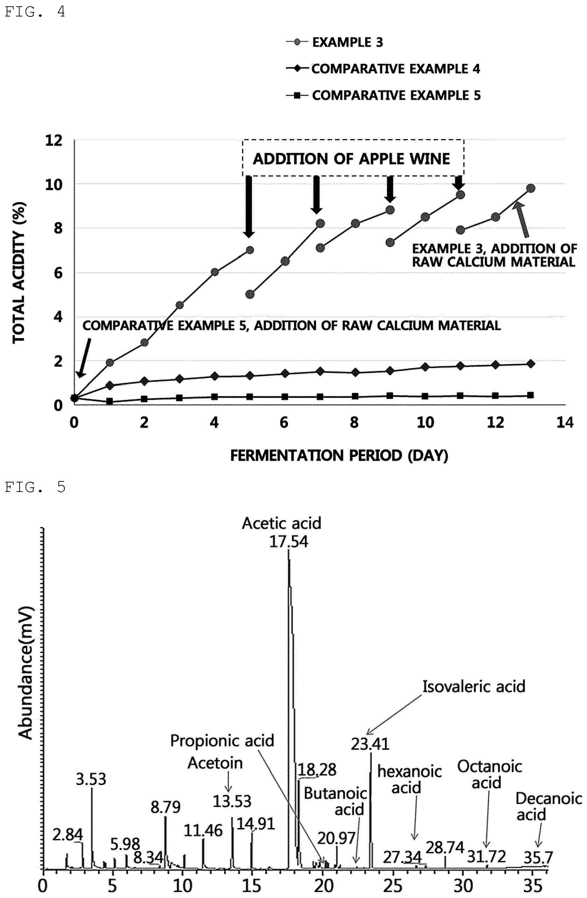

(2) Changes in alcohol production during alcohol fermentation are illustrated in FIG. 3.
(3) Changes in total acidity during acetic acid fermentation are illustrated in FIG. 4.
(4) The results of the experiments are described as follows. In Comparative example 4, 32% of the raw calcium material was dissolved, and thus the loss of the raw calcium material was large. Further, the fermentation yield of alcohol fermentation decreased significantly compared with those of Comparative example 5 and Example 3 in which the alcohol fermentation was carried out without the addition of raw calcium material, so that it was not suitable for the preparation of fermented apple products, indicating that the time of adding was not proper. Further, in the case of Comparative example 5 in which raw calcium material was added at the beginning of the acetic acid fermentation, 86% of the raw calcium material was dissolved and 14.1% of the raw material was lost. In addition, acetic acid fermentation hardly occurred so that it was not suitable for the preparation of fermented apple products, indicating that the time of adding was not proper. In the case of Example 3, 99% of the raw calcium material was dissolved, and the loss of the raw calcium material was small, and it did not affect largely on the alcohol fermentation or acetic acid fermentation yield. It was confirmed that raw calcium material added at the time when the acetic acid fermentation proceeded and the organic acid content was 10,000 ppm or higher is desirable for increasing calcium ion content.

[Experimental example 3] Variation of flavor pattern according to fresh fermentation process

In order to confirm the flavor patterns of Comparative example 2 and Examples 1 and 3, flavor analysis was performed. Each substance was analyzed using GC/MS device by Agilent. CAR/PDMS adsorption was carried out at 40°C for 30 minutes to perform the GC-MS analysis. Other performance conditions are shown in the following Table 3.

[Table 3]

| Injector | 250 |
|---|---|
| Column | DB-WAX |
| Gas Flow rate | 0.8mL/min |
| Oven temp, | 40°C (3min) → 5°C/min → 200°C (10min) |

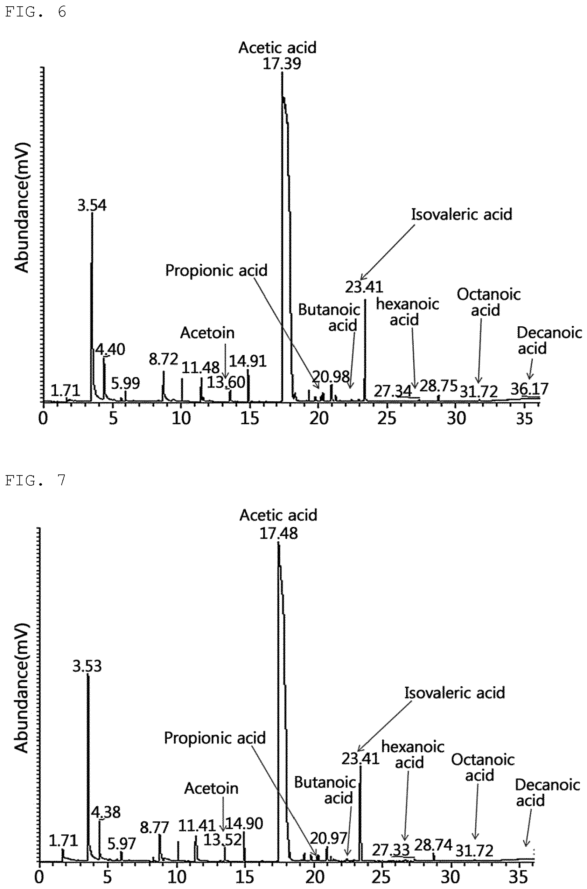

The results of the experiments are shown in Table 4 and FIGS. 5 to 7. FIG. 5 illustrates the results of Comparative example 2. FIG. 6 illustrates the results of Example 1. FIG. 7 illustrates the results of Example 3.

[Table 4]

| Compounds | Odor description | Unit: peak area value | | |
|---|---|---|---|---|
| | | Comparative example 2 | Example 1 | Example 3 |
| Ethyl acetate | Ethereal, fruity | 2,585,317,4 88 | 6,861,523,031 | 6,676,821,504 |
| 2-Butanone | Sweet, acetone-like | 26, 337,743 | - | - |
| Ethanol | - | 220,813,622 | 1,404,484,448 | 1,248,094,158 |
| 2,3-Butanedione | Strong, buttery odor | 409,478,071 | 84,713,858 | 88,777,934 |
| Isobutyl acetate | Fruity, banana | 538,871,281 | 354,603,350 | 324,864,878 |
| 2-Butenal | Spicy buttery odor | 27,780,026 | 40,547,122 | 29,927,955 |
| Ethyl 3-methylbutanoate | Strong, fruity apple | 46,101,889 | 26,942,707 | 22,317,185 |
| 2-Methyl-1-propanol | Fruity, wine-like | 112,589,271 | 77,859,368 | 154,119,489 |
| Isoamyl acetate | sweet, Fruity, banana | 2, 506, 059, 3 30 | 1,544,973,357 | 1,431,775,812 |
| 2-Methylbutyl acetate | Ethereal, banana, apple fruity | - | 206,528,820 | 168, 306,562 |
| 2-Methyl-1-butanol | - | - | 1,351,893,454 | 1,459,839,012 |
| 3-Methyl-1-butanol | Breathtaking , alcoholic | 1,573,165,9 87 | - | - |
| Acetic anhydride | - | 24,896,895 | - | - |
| **Acetoin** | **Slightly sour, yogurt-like, buttery, fatty** | **2,090,685,263** | **635,769,739** | **535,447,884** |
| 3-Oxo-2-butanyl acetate | - | 98,928,125 | - | - |
| Acetic acid | Sour, vinegar | 70,719,447,533 | 107,385,441,4 46 | 80,114,422,322 |
| 2-Furaldehyde | Sweet, caramel-like | 3,922,359,6 84 | 276,746,052 | 281,844,530 |
| 2-Ethyl-1-hexanol | Mild, oily, sweet | - | 35,297,951 | 50,375,932 |
| Benzaldehyde | Cherry, almond-like | 467,046,186 | 229,158,828 | 265,629,493 |
| **Propionic acid** | **Pungent, sour milk** | **336,795,849** | **221,332,270** | **177,670,496** |
| 2,3-Butanediol | - | 212,536,944 | 328,924,423 | 248,297,785 |
| Isopropylformic acid | Sour cheesy | 870,281,494 | 711,728,536 | 578,343,524 |
| 1,2-Propanediol | Odorless | - | 46,238,722 | 38,989,841 |
| Butyrolactone | Slightly caramel-like | - | 31,253,262 | 33,370,150 |
| **Butanoic acid** | **Strong, cheesy** | **109,850,079** | **99,075,618** | **90,308,089** |
| **Isovaleric acid** | **Very sour, sweaty, cheesy, unpleasant odor** | **4,620,296,434** | **4,240,172,396** | **3,896,862,526** |
| 2-Phenylethyl acetate | Sweet, rose | 115,286,246 | 28,346,487 | 52,082,005 |
| **Hexanoic acid** | **Rancid, fatty** | **122,597,444** | **80,583,230** | **93,845,519** |
| 2-Phenyl ethanol | Floral, rose-like | 445,553,626 | 219,500,435 | 263,322,616 |
| **Octanoic acid** | **Fatty, rancid** | **104,762,671** | **51,556,990** | **64,692,378** |

(continued)

| Compounds | Odor description | Unit: peak area value | | |
|---|---|---|---|---|
| | | Comparative example 2 | Example 1 | Example 3 |
| Decanoic acid | Fatty-waxy, cheesy | 30,114,000 | - | - |

The analysis results of the flavor indicated that Examples 1 and 3 prepared by the non-heat sterilization method showed a tendency of decreases in contents of acetoin, propionic acid, butanoic acid, isovaleric acid, hexanoic acid, octanoic acid, and decanoic acid, which are components having the characteristics of the offensive or unpleasant odor (fatty, cheese, unpleasant), compared to Comparative example 2 in which the fermented apple product was prepared by the heat-sterilization treatment of the conventional process. In particular, the results showed decreases of butanoic acid and isovaleric acid, which are the flavor components derived from amino acid, which was one of the components prepared by autolysis and heat-treatment of yeast and decreases of hexanoic acid, octanoic acid, and decanoic acid, which are the flavor components derived from a fatty acid. Therefore, it is confirmed that the processes of Examples 1 to 3 of the present invention reduced the odor compared to conventional Comparative example 2.

[Experimental example 4] Flavor sensory test according to fresh fermentation process

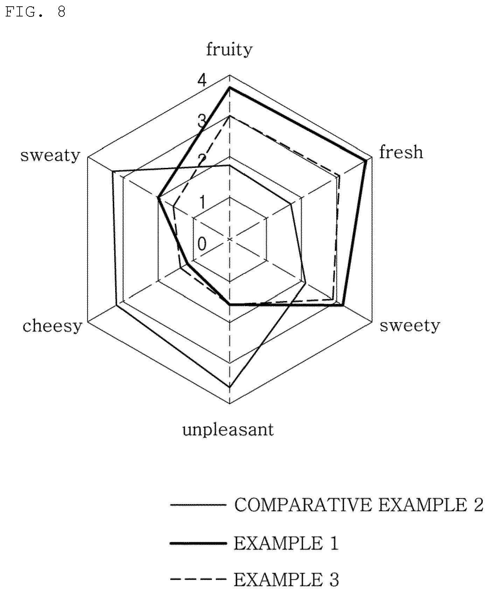

The sensory test was performed to confirm the overall flavor of Comparative example 2 and Examples 1 and 3.
A sensory test was conducted on 20 people aged 20 to 50 in the food industry. The sensory test was carried out on a 5-point scale for each flavor characteristic. For fruity, fresh, sweety, unpleasant, cheesy, and sweaty, 5 points indicate "strong," 4 points indicate "slightly strong," 3 points indicate "average," 2 point indicates "slightly weak," and 1 point indicates "weak."
The results of sensory tests are shown in Table 5 and illustrated in FIG. 8.

[Table 5]

| | Comparative example 2 | Example 1 | Example 3 |
|---|---|---|---|
| Fruity | 1.8 | 3.7 | 3.0 |
| Fresh | 1.7 | 3.8 | 3.1 |
| Sweety | 2.1 | 3.2 | 2.9 |
| Unpleasant | 3.6 | 1.6 | 1.6 |
| Cheesy | 3.2 | 1.2 | 1.4 |
| Sweaty | 3.3 | 2.0 | 1.6 |

Experimental results show that Example 1 has 3 points or more for fruity, fresh and sweety and 2 points or less for unpleasant, cheesy and sweaty, indicating superior sensory effects in the flavor portion compared to those of the Comparative example 2.

[Experimental example 5] Analysis of free sugar and organic acid of fermented apple product

The analysis was performed on free sugars and organic acids contained in the raw apple material of Comparative example 1 and the fermented apple product of Examples 1 and 3, respectively.
(1) Analysis of free sugars was performed by the following method. HPLC analysis was used for analysis of free sugar. For sample pretreatment, 10 ml of liquid was sufficiently dissolved in 50 ml of water, the mixture was constant-volumized using acetonitrile and filtered through a 0.2 μm filter, followed by the analysis. Standard reagents were glucose, fructose, sucrose, lactose, and maltose. The HPLC analysis conditions are shown in the following Table 6.

[Table 6]

| Item | Condition |
|---|---|
| Injection amount | 30 μl |

(continued)

| Item | Condition |
|---|---|
| Detector | RI detector |
| Column temperature | 40°C |
| Moving phase | water 25%, ACN 75% |
| Flow rate | 0.8 mL/min |
| Column | Shodex asahi pak NH2-50 4E |

(2) Analysis of organic acids was performed by the following method. HPLC analysis was used for analysis of organic acid. Sample pretreatment was performed by filtering the liquid sample using a 0.2 $\mu$m filter, followed by the analysis. Standard reagents were citric acid, malic acid, succinic acid, lactic acid, acetic acid, levulinic acid, and pyroglutamic acid. The HPLC analysis conditions are shown in the following Table 7.

[Table 7]

| Item | Condition |
|---|---|
| Injection amount | 30 $\mu$l |
| Detector | RI detector |
| Column temperature | 40°C |
| Moving phase | 6mM perchloric acid |
| Flow rate | 0.8 mL/min |
| Column | Shodex RS pak kc-811 |

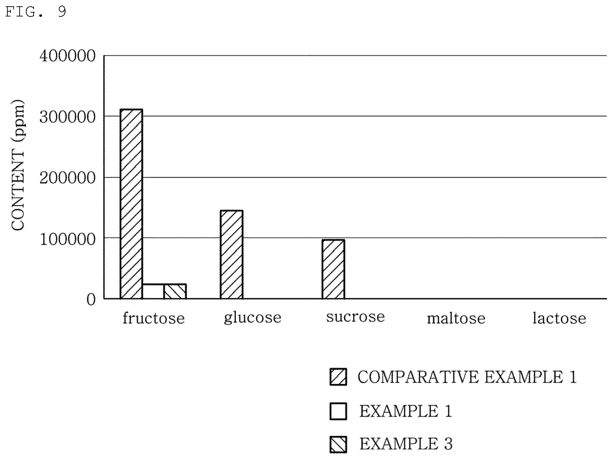

As a result of the experiment, the analysis results of free sugar are illustrated in FIG 9, and the analysis results of organic acid are shown in Table 8.

[Table 8]

| Item | Comparative example 1 (apple concentrate) | Example 1 (fermented apple product) | Example 3 (fermented apple product + calcium hydroxide) |
|---|---|---|---|
| Total content of organic acid (ppm) | 0 | 164,350 | 153, 222 |

As a result of experiments, the content of fructose, glucose, and sucrose was reduced and organic acids were produced in Example 1 and Example 3, which were prepared through fermentation process compared with Comparative example 1. Accordingly, it was confirmed that the fermentation process of Example 1 of the present invention allowed the sugar contained in the raw material to be converted into the organic acid.

Hereinbefore, specific portions of the present invention are described in detail. However, it is apparent by those skilled in the art that these specific Examples are merely preferred embodiments and that the scope of the present invention is not limited thereto. Accordingly, the substantive scope of the present invention is defined by the appended claims and their equivalents.

[Accession number]

Name of depositary institute: The Korean Collection for Type Cultures of the Korea Research Institute of Bioscience and Biotechnology
Accession number: KCTC12749BP
Accession date: January 22, 2015

**Claims**

1. A fermented fruit product containing organic acid, wherein $Ca^{2+}$ ions are contained in an amount of 100 ppm or higher on the basis of the total weight of the fermented fruit product.

2. The fermented fruit product according to claim 1, wherein the fermented fruit product is a fermented product of a fruit concentrate having a sugar content of 10 brix to 50 brix.

3. The fermented fruit product according to claim 1, wherein the fruit comprises an apple.

4. The fermented fruit product according to claim 3, wherein the fruit is an apple.

5. The fermented fruit product according to claim 1, wherein the fermented fruit product is by an alcohol fermention and and acetic acid fermention.

6. The fermented fruit product according to claim 5, wherein the fermented fruit product is a fermented product by *Saccharomyces* genus yeast and *Acetobacter* genus acetic acid bacteria.

7. The fermented fruit product according to claim 1, wherein the organic acid is contained in an amount of 10,000 ppm or higher on the basis of the total weight of the fermented fruit product and a free sugar is contained in an amount of 25,000 ppm or lower on the basis of the total weight of the fermented fruit product.

8. A composition comprising the fermented fruit product according to any one of claims 1 to 7, wherein the composition is a food or a cosmetic composition.

9. A method of preparing the fermented fruit product according to any one of claims 1 to 7, the method comprising:

    alcohol-fermenting a raw fruit material with yeast; and
    acetic acid-fermenting the alcohol-fermented product with acetic acid bacteria to obtain a fermented fruit product,

    wherein a raw calcium material is added at a concentration of 0.05% by weight (w/v) or more on the basis of the total volume of the alcohol fermented product during the acetic acid fermentation, and
    wherein, during the acetic acid fermentation, the raw calcium material is added at the time when a content of the organic acid reaches 10,000 ppm or higher in the fermented product.

10. The method according to claim 9, wherein the method further comprises removing the yeast by non-heat sterilization after completion of the alcohol fermentation.

11. The method according to claim 10, wherein the non-heat sterilization comprises one or more selected from the group consisting of membrane filtration sterilization, high voltage pulsed magnetic field sterilization, ultrahigh pressure sterilization, and ultrasonic sterilization.

12. The method according to claim 9, wherein the removing the yeast is carried out at a temperature of 10°C to 40°C.

13. The method according to claim 9, wherein the alcohol-fermenting comprises repeating addition of 3% by weight to 7% by weight of the raw fruit material on the basis of the total weight of the alcohol-fermented product two times or more after the addition when a alcohol content of the alcohol-fermented product reaches 12% or more to ferment the alcohol-fermented product until the final alcohol content of the alcohol-fermented product reaches 15% or more.

14. The method according to claim 9, wherein the acetic acid-fermenting comprises repeating addition of 15% by weight to 20% by weight of the alcohol-fermented product on the basis of the total weight of the acetic acid-fermented product two times or more after the addition when a acidity of the acetic acid-fermented product reaches 5% or more to ferment the acetic acid-fermented product until the final acidity of the acetic acid-fermented product reaches 9% or more.

15. The method according to claim 9, wherein, during the acetic acid fermentation, the raw calcium material is added at the time when a content of a organic acid in the fermented product is 70,000 ppm to 100,000 ppm.

16. The method according to claim 9, wherein the raw calcium material comprises one or more selected from the group consisting of calcium citrate, calcium gluconate, calcium 5-ribonucleotide, calcium hydroxide, calcium stearyl lactate, calcium chloride, calcium disodium EDTA, calcium lactate, Calcium hydroxyapatite, Calcium hydrogen phosphate, Calcium dihydrogen phosphate, carboxymethylcellulose calcium, calcium carbonate, calcium pantothenate, calcium propionate, calcium sulfate, calcium glycerophosphate, calcium oxide, calcium ascorbate, calcium alginate, calcium acetate, calcium benzoate, calcium stearate, calcium sorbate, calcium ferrocyanide, and calcium caseinate.

17. The method according to claim 16, wherein the calcium carbonate comprises one or more selected from the group consisting of eggshell calcium, shell calcium, seaweed calcium, and pearl calcium.

18. The method according to claim 9, wherein the method further comprises lactic acid-fermentating by lactic acid bacteria before the alcohol fermentation.

19. The method according to claim 18, wherein the yeast is *Saccharomyces* genus, the acetic acid bacteria is *Acetobacter* genus, and the lactic acid bacteria is *Lactobacillus* genus.

20. The method according to claim 19, wherein the *Saccharomyces* genus is *Saccharomyces cerevisiae,* the *Acetobacter* genus is *Acetobacter aceti,* and the *Lactobacillus* genus is *Lactobacillus casei.*

FIG. 1

```
          ┌─────────────────────┐
          │     RAW APPLE       │
          │     MATERIAL        │
          └─────────────────────┘
                    │
                    ▼
          ┌ ─ ─ ─ ─ ─ ─ ─ ─ ─ ─ ┐
            FERMENTATION
          │ BY LACTIC ACID      │
              BACTERIA
          └ ─ ─ ─ ─ ─ ─ ─ ─ ─ ─ ┘
                    │
                    ▼
          ┌─────────────────────┐
          │     ALCOHOL         │
          │   FERMENTATION      │
          └─────────────────────┘
                    │
                    ▼
          ┌─────────────────────┐
          │    ERADICATION      │
          │    OF BACTERIA      │
          └─────────────────────┘
                    │
                    ▼
          ┌─────────────────────┐
          │    ACETIC ACID      │
          │   FERMENTATION      │
          └─────────────────────┘
┌──────────────┐            │
│ RAW CALCIUM  │            │
│  MATERIAL    │────────────▶
└──────────────┘    ┌─────────────────────┐
                    │       AGING         │
                    └─────────────────────┘
                              │
                              ▼
                    ┌─────────────────────┐
                    │  FERMENTED APPLE    │
                    │ PRODUCT CONTAINING  │
                    │ GREAT QUANTITY OF   │
                    │    CALCIUM ION      │
                    └─────────────────────┘
```

18

FIG. 2

FIG. 3.

FIG. 4

FIG. 5

FIG. 6

FIG. 7

FIG. 8

COMPARATIVE EXAMPLE 2

EXAMPLE 1

EXAMPLE 3

FIG. 9

## INTERNATIONAL SEARCH REPORT

| International application No. |
| --- |
| PCT/KR2017/004609 |

**A. CLASSIFICATION OF SUBJECT MATTER**

*A23L 33/105(2016.01)i, C12J 1/04(2006.01)i, A61K 8/97(2006.01)i, A61Q 19/00(2006.01)i, A61Q 19/08(2006.01)i*

According to International Patent Classification (IPC) or to both national classification and IPC

**B. FIELDS SEARCHED**

Minimum documentation searched (classification system followed by classification symbols)
A23L 33/105; A23L 1/304; C12J 1/00; A23L 2/06; C12J 1/04; A23L 2/02; A61K 8/97; A61Q 19/00; A61Q 19/08

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: fruit fermented product, organic acid, Ca2+, alcohol fermentation, acetic acid fermentation

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2002-0091321 A (CHOI, Hyuk Mun) 06 December 2002 See page 2, lines 23-34; page 3, lines 14-15; example 2; claims 1, 9; and tables 1, 3. | 1-8 |
| A | | 9-20 |
| A | KR 10-2010-0008231 A (DAESANG CORPORATION) 25 January 2010 See paragraphs [0018], [0022], [0041]; claims 1, 2, 6; and figure 5. | 1-20 |
| A | JP 11-103821 A (KONDO, Takashi et al.) 20 April 1999 See paragraphs [0003], [0010], [0013], [0014], [0017]; and claim 1. | 1-20 |
| A | KR 10-2012-0060697 A (WOONGJIN FOODS CO., LTD.) 12 June 2012 See paragraphs [0018], [0024]; and claims 1-4, 11. | 1-20 |
| A | KR 10-2004-0042237 A (KEIMYUUNG FOODEX CO., LTD.) 20 May 2004 See page 3, lines 8-10; examples 1, 3; claims 1-5; and table 1, | 1-20 |

☐ Further documents are listed in the continuation of Box C.  ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 08 AUGUST 2017 (08.08.2017) | **08 AUGUST 2017 (08.08.2017)** |

| Name and mailing address of the ISA/KR | Authorized officer |
| --- | --- |
| Korean Intellectual Property Office Government Complex-Daejeon, 189 Seonsa-ro, Daejeon 302-701, Republic of Korea Facsimile No. +82-42-481-8578 | Telephone No. |

Form PCT/ISA/210 (second sheet) (January 2015)

**INTERNATIONAL SEARCH REPORT**
Information on patent family members

International application No.

**PCT/KR2017/004609**

| Patent document cited in search report | Publication date | Patent family member | Publication date |
|---|---|---|---|
| KR 10-2002-0091321 A | 06/12/2002 | NONE | |
| KR 10-2010-0008231 A | 25/01/2010 | KR 10-1406739 B1 | 13/06/2014 |
| JP 11-103821 A | 20/04/1999 | JP 3308475 B2 | 29/07/2002 |
| KR 10-2012-0060697 A | 12/06/2012 | KR 10-1175038 B1 | 17/08/2012 |
| KR 10-2004-0042237 A | 20/05/2004 | KR 10-0499619 B1 | 05/07/2005 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101321611

**Non-patent literature cited in the description**

- **HYESOO KIM et al.** *J Nutr Health,* 2014, vol. 47 (2), 134-144

- **P. COMUZZO et al.** *Food chemistry,* 2006, vol. 99, 217-230